# FASCICULE DE BREVET EUROPEEN

(11) **EP 0 998 905 B1**
(45) Date de publication et mention de la délivrance du brevet: **07.11.2001**
(21) Numéro de dépôt: 99401807.5
(22) Date de dépôt: 19.07.1999
(51) Int. Cl.: A61K 7/032

(54) **Composition de maquillage comprenant une poly-alpha-olefine**
Schminkzusammensetzung enthaltend Polyalphaolefinen
Make-up composition containing a poly-alpha-olefin

(30) Priorité: 25.09.1998 FR 9812043
(43) Date de publication de la demande: 10.05.2000
(73) Titulaire: L'OREAL, 75008 Paris (FR)
(72) Inventeur: Collin, Nathalie, 92330 Sceaux (FR)
(74) Mandataire: Lhoste, Catherine

(56) Documents cités:
- EP-A- 0 685 221
- EP-A- 0 792 633
- EP-A- 0 815 826
- WO-A-91/12793
- WO-A-95/15741
- WO-A-97/35542
- US-A- 4 060 569
- US-A- 4 239 546
- US-A- 5 725 845

## Description

La présente invention concerne une composition pour le maquillage des fibres kératiniques, notamment des cils et des cheveux d'être humains, comprenant une cire de polyoléfine. L'invention se rapporte également à l'utilisation de cette composition pour le maquillage des fibres kératiniques, ainsi qu'un procédé de maquillage de ces dernières. La composition et le procédé de maquillage selon l'invention sont plus particulièrement destinés aux fibres kératiniques sensiblement longitudinales telles que les cils, les sourcils et les cheveux, y compris les faux-cils et les postiches. Plus spécialement, l'invention porte sur un mascara.

Les mascaras sont couramments préparés selon deux types de formulations : les mascaras aqueux, dits mascaras crèmes, sous forme d'émulsion de cires dans l'eau ; les mascaras anhydres ou à faible teneur en eau, dits mascaras waterproofs, sous forme de dispersions de cires dans des solvants.

Il est connu d'employer diverses cires pour la formulation des mascaras comme celles décrites dans le document WO-A-91/12793. Ces cires, selon leur nature, confèrent au mascara des propriétés de maquilage différentes. Par exemple, la cire de carnauba confère de la dureté au film déposé sur les cils tandis que la cire d'abeille apporte une bonne adhérence du film sur les cils mais les cils ont alors tendance à se coller.

Pour obtenir les propriétés optimales de maquillage, il est possible d'utiliser des mélanges de cires comme décrit dans le document WO-A-95/15741. Toutefois, les mascaras obtenus ne sont pas toujours faciles à appliquer sur les cils avec les applicateurs courant tels que les brosses de mascara : lors de l'application du mascara sur les cils le contact de la composition avec le cil peut laisser une sensation d'accroche et de frein. De plus, lorsque le mascara comprend des cires trop dures, il est difficile de travailler avec la brosse le dépôt de produit sur les cils ce qui ne permet pas de contrôler le maquillage, ni d'obtenir un gainage homogène des cils ; de'plus, un film rigide de mascara laisse une sensation d'inconfort à l'utilisatrice et a tendance à s'effriter au cours du temps, ce qui est contraire à l'obtention d'un maquillage présentant une bonne tenue.

Le but de la présente invention est de disposer d'une composition de maquillage des matières kératiniques, et notamment des fibres tels que les cils, sous forme d'émulsion cire-dans-eau s'appliquant facilement sur les matières kératiniques et conduisant, après application, à un maquillage homogène, confortable à porter et présentant une bonne tenue.

Les inventeurs ont découvert qu'une telle composition pouvait être obtenue en utilisant une cire de polyoléfine particulière. On obtient alors une composition onctueuse, facile à appliquer sur les matières kératiniques. Les matières kératiniques maquillés sont bien gainées, de façon homogène. De plus, la composition glisse bien sur les cils et est facile à travailler avec la brosse. Le dépôt du produit de maquillage sur les cils, permet ainsi de moduler aisément le maquillage recherché sur les cils et notamment de maîtriser la quantité de produit à déposer sur les cils. En outre, la cire de polyoléfine est bien compatible avec les cires couramment utilisées dans les mascaras, ce qui permet de l'utiliser en mélange avec ces cires courantes sans introduire de contrainte particulière.

La présente invention a donc pour objet une composition de maquillage des fibres kératiniques sous forme d'émulsion de cire-dans-eau, caractérisée par le fait qu'elle comprend au moins une cire de polyoléfine résultant de l'homopolymérisation d'alpha-oléfines ayant au moins 10 atomes de carbone, ladite cire ayant un point de fusion allant de 50 à 80 °C.

L'invention a encore pour objet un produit de mascara comprenant un réservoir, contenant une composition de mascara, et une brosse d'application de la composition définie précédemment sur les fibres kératiniques, notamment les cils.

L'invention a aussi pour objet un procédé de maquillage des fibres kératiniques, notamment des cils, consistant à appliquer sur les matières kératiniques une composition telle que définie précédemment.

L'invention a également pour objet l'utilisation d'une composition telle que définie précédemment pour l'obtention d'un maquillage homogène et/ou facile à appliquer et/ou ayant une bonne tenue.

L'invention a encore pour objet l'utilisation d'une cire de polyoléfine telle que définie précédemment dans une composition de maquillage des fibres kératiniques sous forme d'émulsion cire-dans-eau.

La cire de polyoléfine utilisée dans la composition selon l'invention est issue de l'homopolymérisation d'alpha-oléfine répondant à la formule générale : R-CH=CH₂ dans laquelle R désigne un radical alkyle ayant de 10 à 50 atomes de carbone, et de préférence de 25 à 50 atomes de carbone, ou leurs mélanges. De préférence, R est un radical alkyl linéaire. Selon l'invention, on entend par homopolymérisation d'alpha-oléfine la polymérisation de monomères consistant essentiellement en une alpha-oléfine ou un mélange d'alpha-oléfines telles que définies précédemment

La cire de polyoléfine a avantageusement une pénétrabilité à l'aiguille, mesurée à 44 °C, allant de 15 à 120, de préférence de 100 à 120, et mieux de 105 à 115. La cire de polyoléfine a de préférence un point de fusion allant de 50 °C à 60 °C.

La cire de polyoléfine peut avoir un poids moléculaire moyen en nombre allant de 400 à 3000, de préférence de 2000 à 3000 et mieux de 2500 à 2700.

De telles cires de polyoléfine sont décrites dans les brevets US-A-4060569 et US-A-4239546. Ces cires sont notamment vendues sous la dénomination de "PERFORMA V® 103", "PERFORMA V® 253", "PERFORMA V® 260" par la société PETROLITE.

Ces cires présentent les caractéristiques suivantes :

| | PERFORMA V® 103 | PERFORMA V® 253 | PERFORMA V® 260 |
|---|---|---|---|
| point de fusion (norme ASTM D 36) | 74 °C | 67 °C | 54°C |
| poids moléculaire moyen en nombre : | 2800 | 520 | 2600 |
| polydispersité du poids moléculaire | 6 | 8 | 11,5 |
| densité mesurée à 25 °C (norme ASTM D 792) | 0,92 g/cm³ | 0,92 g/cm³ | 0,90 g/cm³ |
| dureté (pénétrabilité à l'aiguille - norme ASTM D 1321) à 25 °C | 5 | 7 | 12 |
| dureté (pénétrabilité à l'aiguille - norme ASTM D 1321) à 44 °C | 20 | - | 110 |
| viscosité à 99°C (norme ASTM D 792) | 0,345 Pa.s (345 centipoises) | 0,006 Pa.s (6 centipoises) | 0,358 Pa.s. (358 centipoises) |

La polydispersité du poids moléculaire correspond au rapport du poids moléculaire moyen en poids sur le poids moléculaire moyen en nombre.

La pénétrabilité à l'aiguille des cires est déterminée selon la norme française NF T 60-123 ou la norme américaine ASTM D 1321, à la température de 44 °C. Selon ces normes, la pénétrabilité à l'aiguille est la mesure de la profondeur, exprimée en dixièmes de millimètre, à laquelle une aiguille normalisée, pesant 2,5 g disposée dans un équipage mobile pesant 97,5 g et placée sur la cire à tester, pendant 5 secondes, pénètre dans la cire.

La cire de polyoléfine a de préférence une densité allant de 0,85 à 0,95 g/cm³.

La cire de polyoléfine peut être présente, dans la composition selon l'invention, en une teneur allant de 0,1 % à 20% en poids, par rapport au poids total de la composition, et de préférence de 0,5 % à 12 % en poids.

La composition selon l'invention peut contenir, outre la cire de polyoléfine définie précédemment, une ou plusieurs cires additionnelles qui peuvent être choisies parmi les cires d'origine animale, les cires d'origine végétale ou les cires d'origine synthétique.

Les cires additionnelles susceptibles d'être utilisées dans la composition selon l'invention possèdent en règle générale un point de fusion compris entre 40 et 110 °C et ont une pénétrabilité à l'aiguille allant de 1 à 217. La pénétrabilité à l'aiguille des cires est déterminée selon la norme française NF T 60-123 ou la norme américaine ASTM D 1321, à la température de 25 °C. Selon ces normes, la pénétrabilité à l'aiguille est la mesure de la profondeur, exprimée en dixièmes de millimètre, à laquelle une aiguille normalisée, pesant 2,5 g disposée dans un équipage mobile pesant 97,5 g et placée sur la cire à tester, pendant 5 secondes, pénètre dans la cire.

Parmi les cires d'origine animale, on peut citer les cires d'abeille, les cires de lanoline et les cires d'insectes de Chine.
Parmi les cires d'origine végétale, on peut citer les cires de riz, les cires de Carnauba, de Candellila, d'Ouricurry, les cires de fibres de liège, les cires de canne à sucre, les cires du Japon, la cire de Sumac, la cire de coton.
Parmi les cires d'origine minérale, on peut citer les paraffines, les cires microcristallines, les cires de Montan et les ozokérites.
Parmi les cires d'origine synthétique, on peut utiliser notamment les cires de polyéthylène, les cires obtenues par la synthèse de Fischer et Tropsch, les copolymères cireux ainsi que leurs esters, les cires de silicone.
Il est également possible d'utiliser des huiles d'origine animales ou végétales hydrogénées qui répondent toujours aux deux caractéristiques physiques mentionnées précédemment.
Parmi ces huiles, on peut citer les cires de jojoba hydrogénées et les huiles hydrogénées qui sont obtenues par hydrogénation catalytique de corps gras composés de chaîne grasse linéaire ou non en C₈-C₃₂, l'huile de tournesol hydrogénée, l'huile de ricin hydrogénée, l'huile de coprah hydrogénée, la lanoline hydrogénée et les huiles de palme hydrogénées.
Les cires utilisables selon la présente invention sont de préférence solides et rigides à température inférieure à 50 °C.

La composition selon l'invention peut comprendre de 0,1 % à 30 % en poids de cire additionnelle, en poids par rapport au poids total de la composition, de préférence de 1 % à 20 % en poids.

De préférence, la composition selon l'invention peut comprendre :
- au moins une cire additionnelle ayant une pénétrabilité à l'aiguille allant de 1 à 7, 5 (dite cire I), notamment en une teneur allant de 0,1 % à 20 % en poids par rapport au poids total de la composition, et
- au moins une cire additionnelle ayant une pénétrabilité à l'aiguille supérieure à 7,5 et inférieure ou égale à 217 (dite cire II), notamment en une teneur allant de 0,1 % à 10 % en poids par rapport au poids total de la composition.

Selon l'invention, les cires peuvent être présentes dans la composition sous forme de particules ayant une taille allant de 50 nm à 10 µm, et de préférence de 50 nm à 3,5 µm.

La teneur en eau dans la composition selon l'invention peut aller de 20 % à 99 % en poids, de préférence de 50 % à 80 % en poids, par rapport au poids total de la composition.

La composition selon l'invention peut contenir, en outre, un polymère filmogène qui peut être solubilisé et/ou sous forme de particules en dispersion dans la phase aqueuse.

Le polymère filmogène peut être choisi parmi :
- les dérivés de kératine, tels que les hydrolysats de kératine et les kératines sulfoniques ;
- les dérivés de chitine ou de chitosane anioniques, cationiques, amphotères ou non-ioniques ;
- les dérivés de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates;
- les polyvinylpyrrolidones et les copolymères vinyliques, tels que les copolymères de l'éther méthylvinylique et de l'anhydride malique, ou le copolymère de l'acétate de vinyle et de l'acide crotonique ;
- les polymères polyester et/ou polyesteramide anioniques dispersables dans l'eau, comprenant des monomères portant une fonction : -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut citer en particulier les polymères décrits dans les documents US-3,734,874 ; US-4,233,196; US-4,304,901. Avantageusement, on choisit des polymères polyesters filmogènes à base d'au moins un acide dicarboxylique, d'au moins un diol et d'au moins un monomère aromatique bifonctionnel portant en outre un groupement - SO₃M tel que décrit ci-dessus.
- les polyesters à chaîne grasse, les polyamides, et les résines époxyesters.
- les polymères polyuréthannes, notamment les polyuréthannes anioniques, cationiques, non ioniques ou amphotères, les polyuréthannes-acryliques, les polyuréthannes-polyvinylpirrolidones, les polyester-polyuréthannes, les polyéther-polyuréthannes, les polyurées, les polyurée/polyuréthannes, et leurs mélanges.
- les polymères d'origine naturelle, éventuellement modifiés, tels que :
   . les gommes arabiques, la gomme de guar, les dérivés du xanthane, la gomme de karaya ;
   . les alginates et les carraghénates ;
   . les glycoaminoglycanes, l'acide hyaluronique et ses dérivés ;
   . la résine shellac, la gomme de sandaraque, les dammars, les élémis, les copals.

Le polymère filmogène peut être présent dans la composition en une teneur en matière sèche allant de 0,1 % à 10 % en poids, par rapport au poids total de la composition.

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans les compositions selon l'invention sont:
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés.
- parmi les tensioactifs anioniques : les acides gras en C16-C30 neutralisés par les amines, l'ammoniaque ou les sels alcalins.

On utilise de préférence des tensioactifs permettant l'obtention d'émulsion huile-dans-eau.

En outre, la composition peut comprendre au moins un agent épaississant, de préférence de nature hydrophile. Celui-ci peut par exemple être choisi parmi les polymères carboxyvinyliques (carbomer), les copolymères acryliques tels que les copolymères d'acrylates/alkylacrylates, les polyacrylamides, les polysaccharides, les gommes naturelles et les argiles.

La composition selon l'invention peut également contenir des ingrédients couramment utilisés en cosmétique, tels que les vitamines, les oligo-éléments, les adoucissants, les séquestrants, les parfums, les huiles, les silicones, les vitamines, les protéines, les céramides, les plastifiants, les agents de cohésion ainsi que les agents alcalinisants ou acidifiants habituellement utilisés dans le domaine cosmétique, les charges, les pigments, les émollients, les conservateurs.

En particulier, lorsque la composition comprend la cire de polyoléfine "PERFORMA V® 103", la composition ne comprend pas les ingrédients suivants, selon les teneurs indiquées :
- 1 g de cire de polyoléfine "PERFORMA V® 103",
- 1,5 g de copolymère polyvinylpyrrolidone/eicosène
- 2 g de butylène glycol,
- 0,35 g d'hydroxyéthylcellulose,
- 4 g de stéarate de glycéryle
- 4,3 g de cire d'abeille,
- 3,1 g de cire de camauba.

La composition selon l'invention est destinée à un produit de mascara comprenant un réservoir, contenant ladite composition de mascara, et un système d'application de ladite composition sur les fibres kératiniques, notamment les cils. Le réservoir est muni de façon connue d'une ouverture dans laquelle est logée un système d'essorage. Le système d'application comporte une tige munie à une première extrémité d'une brosse et à une deuxième extrémité d'un bouchon destiné à fermer le réservoir. Un tel conditionnement est notamment illustré à la figure 7 de la demande EP-A-611170.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition selon l'invention peut être préparée selon les méthodes usuelles des domaines considérés.

L'invention est illustrée plus en détail dans les exemples suivants.

### Exemple 1 :

On a préparé un mascara ayant la composition suivante :
- Cire d'abeille 3,6 g
- Cire de carnauba 2,9 g
- Pigments 5,5 g
- Agent épaississant 4,6 g
- Cire de polyoléfine (PERFORMA V® 260 de PETROLITE) 11,4 g
- Acide stéarique 5,8 g
- Triéthanolamine 2,4 g
- amino-2 méthyl-2 propanediol-1,3 0,5 g
- D-panthénol 0,5 g
- Conservateurs qs
- Eau qsp 100 g

On obtient un mascara de consistance crémeuse qui s'étale bien sur les cils et qui est facile à travailler avec la brosse, permettant ainsi d'obtenir un maquillage homogène et gainant parfaitement les cils.

### Exemple 2 :

On a préparé un mascara ayant la composition suivante :
- Cire d'abeille 4,7 g
- Cire de camauba 3,7 g
- Cire de paraffine 12,8 g
- Cire de polyoléfine (PERFORMA V® 260 de PETROLITE) 0,5 g
- Agent épaississant 4,6 g
- Pigments 7,1 g
- Acide stéarique 7,1 g
- Triéthanolamine 3,7 g
- D-panthénol 0,5 g
- Mélange de diméthiconol et de décaméthyl pentasiloxane (15/85) (DC2-9071 de DOW CORNING) 5 g
- Eau qsp 100 g

Le mascara s'applique facilement sur les cils avec une bonne onctuosité et enrobe parfaitement les cils.

## Revendications

1. Composition de maquillage des matières kératiniques sous forme d'émulsion de cire-dans-eau, **caractérisée par le fait qu'**elle comprend au moins une cire de polyoléfine résultant de l'homopolymérisation d'alpha-oléfines ayant au moins 10 atomes de carbone, ladite cire ayant un point de fusion allant de 50 à 80 °C.

2. Composition selon la revendication 1, **caractérisée par le fait que** la cire de polyoléfine a un point de fusion allant de 50 °C à 60 °C.

3. Composition selon la revendication 1 ou 2, **caractérisée par le fait que** la cire de polyoléfine a une pénétrabilité à l'aiguille, mesurée à 44 °C, allant de 15 à 120.

4. Composition selon l'une quelconque des revendications précédentes, **caractérisée par le fait que** la cire de polyoléfine a une pénétrabilité à l'aiguille, mesurée à 44 °C, allant de 100 à 120, et mieux de 105 à 115.

5. Composition selon l'une quelconque des revendications précédentes, **caractérisée par le fait que** ladite cire de polyoléfine à un poids moléculaire moyen en nombre allant de 400 à 3000.

6. Composition selon l'une quelconque des revendications précédentes, **caractérisée par le fait que** ladite cire de polyoléfine à un poids moléculaire moyen en nombre allant de 2000 à 3000, et mieux de 2500 à 2700.

7. Composition selon l'une quelconque des revendications précédentes, **caractérisée par le fait que** ladite cire de polyoléfine a une densité allant de 0,85 à 0,95 g/cm³.

8. Composition selon l'une quelconque des revendications précédentes, **caractérisée par le fait que** la cire de polyoléfine résulte de l'homopolymérisation d'alpha-oléfine répondant à la formule générale : R-CH=CH₂ dans laquelle R désigne un radical alkyle ayant de 10 à 50 atomes de carbone ou leur mélange.

9. Composition selon la revendication 8, **caractérisée par le fait que** R désigne un radical alkyle a de 25 à 50 atomes de carbone.

10. Composition selon l'une des revendications 8 ou 9, **caractérisée par le fait que** le radical alkyl R est linéaire.

11. Composition selon l'une quelconque des revendications précédentes, **caractérisée par le fait que** la cire de polyoléfine est présente en une teneur allant de 0,1 % à 20% en poids, par rapport au poids total de la composition.

12. Composition selon l'une quelconque des revendications précédentes, **caractérisée par le fait que** la cire de polyoléfine est présente en une teneur allant de 0,5 % à 12 % en poids, par rapport au poids total de la composition.

13. Composition selon l'une quelconque des revendications précédentes, **caractérisée par le fait qu'**elle comprend en outre une ou plusieurs cires additionnelles d'origine animale, végétale ou synthétique.

14. Composition selon l'une quelconque des revendications précédentes, **caractérisée par le fait que** la cire additionnelle a une pénétrabilité à l'aiguille, à 25 °C, allant de 1 à 217.

15. Composition selon l'une quelconque des revendications précédentes, **caractérisée par le fait qu'**elle comprend au moins une cire additionnelle ayant une pénétrabilité à l'aiguille allant de 1 à 7, 5 et au moins une cire additionnelle ayant une pénétrabilité à l'aiguille supérieure à 7,5 et inférieure ou égale à 217, mesurée à 25 °C.

16. Composition selon l'une des revendications 13 à 15, **caractérisée par le fait que** la cire additionnelle est présente en une teneur allant 0,1 % à 30 % en poids, par rapport au poids total de la composition, et mieux de 1 % à 20 % en poids.

17. Composition selon l'une quelconque des revendications précédentes, **caractérisée par le fait qu'**elle comprend, en outre, au moins un polymère filmogène.

18. Composition selon la revendication17, **caractérisée par le fait que** le polymère filmogène est solubilisé ou sous forme de particules en dispersion dans la phase aqueuse de la composition.

19. Composition selon l'une des revendications 17 ou 18, **caractérisée par le fait que** la teneur en polymère filmogène va de 0,1 % à 10 % en poids, par rapport au poids total de la composition.

20. Composition selon l'une quelconque des revendications précédentes, **caractérisée par le fait qu'**elle comprend au moins un tensioactif émulsionnant.

21. Composition selon la revendication 20, **caractérisée par le fait que** le tensioactif émulsionnant est présent en une teneur allant de 2 % à 30 % en poids, par rapport au poids total de la composition.

22. Composition selon l'une quelconque des revendications précédentes, **caractérisée par le fait qu'**elle comprend au moins un agent épaississant.

23. Composition selon l'une quelconque des revendications précédentes, **caractérisée par le fait que** la composition comprend au moins un additif choisi dans le groupe formé par les vitamines, les oligo-éléments, les adoucissants, les séquestrants, les parfums, les huiles, les silicones, les protéines, les céramides, les plastifiants, les agents de cohésion, les agents alcalinisants ou acidifiants, les charges, les pigments, les émollients, les conservateurs.

24. Composition selon l'une quelconque des revendications précédentes, **caractérisée par le fait que** la teneur en eau va de 20 % à 99 % en poids, de préférence de 50 % à 80 % en poids, par rapport au poids total de la composition.

25. Produit de mascara comprenant un réservoir, contenant une composition de mascara, et une brosse d'application de ladite composition sur les fibres kératiniques, notamment les cils, **caractérisé par le fait que** la composition est une composition selon l'une quelconque des revendications 1 à 24.

26. Procédé de maquillage des matières kératiniques, notamment des cils, **caractérisé par le fait que** l'on applique sur les fibres kératiniques une composition selon l'une quelconque des revendications 1 à 24.

27. Utilisation d'une composition telle que définie selon l'une quelconque des revendications 1 à 24 pour l'obtention d'un maquillage homogène et/ou facile à appliquer et/ou ayant une bonne tenue.

28. Utilisation d'une cire de polyoléfine selon l'une des revendications 1 à 12 dans une composition de maquillage des fibres kératiniques sous forme d'émulsion cire-dans-eau.

29. Mascara comprenant une composition selon l'une quelconque des revendications 1 à 24.

## Patentansprüche

1. Zusammensetzung zum Schminken von Keratinfasern in Form einer Wachs-in-Wasser-Emulsion, **dadurch gekennzeichnet, daß** sie mindestens ein Polyolefinwachs enthält, das bei der Homopolymerisation von α-Olefinen mit mindestens 10 Kohlenstoffatomen entsteht, wobei das Wachs einen Schmelzpunkt im Bereich von 50 bis 80 °C aufweist.

2. Zusammensetzung nach Anspruch 1, **dadurch gekennzeichnet, daß** Polyolefinwachs einen Schmelzpunkt im Bereich von 50 bis 60 °C besitzt.

3. Zusammensetzung nach Anspruch 1 oder 2, **dadurch gekennzeichnet, daß** das Polyolefinwachs eine bei 44 °C bestimmte Nadelpenetration von 15 bis 120 aufweist.

4. Zusammensetzung nach einem der vorhergehenden Ansprüche, **dadurch gekennzeichnet, daß** das Polyolefinwachs eine bei 44 °C bestimmte Nadelpenetration von 100 bis 120 und besser 105 bis 115 aufweist.

5. Zusammensetzung nach einem der vorhergehenden Ansprüche, **dadurch gekennzeichnet, daß** das Polyolefinwachs ein Zahlenmittel des Molekulargewichts von 400 bis 3000 aufweist.

6. Zusammensetzung nach einem der vorhergehenden Ansprüche, **dadurch gekennzeichnet, daß** das Polyolefinwachs ein Zahlenmittel des Molekulargewichts von 2000 bis 3000 und besser 2500 bis 2700 hat.

7. Zusammensetzung nach einem der vorhergehenden Ansprüche, **dadurch gekennzeichnet, daß** das Polyolefinwachs eine Dichte von 0,85 bis 0,95 g/cm³ besitzt.

8. Zusammensetzung nach einem der vorhergehenden Ansprüche, **dadurch gekennzeichnet, daß** das Polyolefinwachs aus der Homopolymerisation eines α-Olefins der allgemeinen Formel: R-CH=CH₂ resultiert, wobei R eine Alkylgruppe mit 10 bis 50 Kohlenstoffatomen oder deren Gemische bedeutet.

9. Zusammensetzung nach Anspruch 8, **dadurch gekennzeichnet, daß** R eine Alkylgruppe mit 25 bis 50 Kohlenstoffatomen bedeutet.

10. Zusammensetzung nach Anspruch 8 oder 9, **dadurch gekennzeichnet, daß** die Alkylgruppe R geradkettig ist.

11. Zusammensetzung nach einem der vorhergehenden Ansprüche, **dadurch gekennzeichnet, daß** das Polyolefinwachs in einer Menge von 0,1 bis 20 Gew.-%, bezogen auf das Gesamtgewicht der Zusammensetzung, vorliegt.

12. Zusammensetzung nach einem der vorhergehenden Ansprüche, **dadurch gekennzeichnet, daß** das Polyolefinwachs in einer Menge von 0,5 bis 12 Gew.-%, bezogen auf das Gesamtgewicht der Zusammensetzung, vorliegt.

13. Zusammensetzung nach einem der vorhergehenden Ansprüche, **dadurch gekennzeichnet, daß** sie außerdem ein oder mehrere zusätzliche Wachse tierischer, pflanzlicher oder synthetischer Herkunft enthält.

14. Zusammensetzung nach einem der vorhergehenden Ansprüche, **dadurch gekennzeichnet, daß** das zusätzliche Wachs bei 25 °C eine Nadelpenetration von 1 bis 217 aufweist.

15. Zusammensetzung nach einem der vorhergehenden Ansprüche, **dadurch gekennzeichnet, daß** sie mindestens ein zusätzliches Wachs mit einer Nadelpenetration von 1 bis 7,5 und mindestens ein zusätzliches Wachs mit einer Nadelpenetration größer 7,5 und höchstens 217 enthält.

16. Zusammensetzung nach einem der Ansprüche 13 bis 15, **dadurch gekennzeichnet, daß** das zusätzliche Wachs in einer Menge von 0,1 bis 30 Gew.-%, bezogen auf das Gesamtgewicht der Zusammensetzung, und besser 1 bis 20 Gew.-% vorliegt.

17. Zusammensetzung nach einem der Ansprüche 13 bis 15, **dadurch gekennzeichnet, daß** sie ferner mindestens ein filmbildendes Polymer enthält.

18. Zusammensetzung nach Anspruch 17, **dadurch gekennzeichnet, daß** das filmbildende Polymer in Lösung oder in Form von in der wäßrigen Phase der Zusammensetzung dispergierten Partikeln vorliegt.

19. Zusammensetzung nach einem der Ansprüche 17 oder 18, **dadurch gekennzeichnet, daß** der Mengenanteil des filmbildenden Polymers im Bereich von 0,1 bis 10 Gew.-%, bezogen auf das Gesamtgewicht der Zusammensetzung, liegt.

20. Zusammensetzung nach einem der vorhergehenden Ansprüche, **dadurch gekennzeichnet, daß** sie mindestens einen emulgierenden grenzflächenaktiven Stoff enthält.

21. Zusammensetzung nach Anspruch 20, **dadurch gekennzeichnet, daß** der emulgierende grenzflächenaktive Stoff in einer Menge von 2 bis 30 Gew.-%, bezogen auf das Gesamtgewicht der Zusammensetzung, vorliegt.

22. Zusammensetzung nach einem der vorhergehenden Ansprüche, **dadurch gekennzeichnet, daß** sie mindestens ein Verdickungsmittel enthält.

23. Zusammensetzung nach einem der vorhergehenden Ansprüche, **dadurch gekennzeichnet, daß** sie ferner mindestens einen Zusatzstoff enthält, der unter den Vitaminen, Spurenelementen, reizlindernden Mitteln, Maskierungsmitteln, Parfums, Ölen, Siliconen, Vitaminen, Proteinen, Ceramiden, Weichmachern, Kohäsionsmitteln, Alkalisierungsmitteln, Ansäuerungsmitteln, Füllstoffen, Pigmenten, Emollentien und Konservierungsmitteln ausgewählt ist.

24. Zusammensetzung nach einem der vorhergehenden Ansprüche, **dadurch gekennzeichnet, daß** der Wassergehalt im Bereich von 20 bis 99 Gew.-% und vorzugsweise 50 bis 80 Gew.-%, bezogen auf das Gesamtgewicht der Zusammensetzung, liegt.

25. Mascaraprodukt, das einen Behälter mit einer Mascarazusammensetzung und eine Bürste zum Auftragen der Zusammensetzung auf die Keratinfasern und insbesondere die Wimpern umfaßt, **dadurch gekennzeichnet, daß** die Zusammensetzung eine Zusammensetzung nach einem der Ansprüche 1 bis 24 ist.

26. Verfahren zum Schminken von Keratinfasern und insbesondere Wimpern, **dadurch gekennzeichnet, daß** auf die Keratinfasern eine Zusammensetzung nach einem der Ansprüche 1 bis 24 aufgebracht wird.

27. Verwendung einer Zusammensetzung nach einem der Ansprüche 1 bis 24, um eine Schminke herzustellen, die homogen ist und/oder leicht aufgetragen werden kann und/oder gut haftet.

28. Verwendung eines Polyolefinwachses nach einem der Ansprüche 1 bis 12 in einer Zusammensetzung zum Schminken von Keratinfasern, die in Form einer Wachs-in-Wasser-Emulsion vorliegt.

29. Mascara, die eine Zusammensetzung nach einem der Ansprüche 1 bis 24 enthält.

## Claims

1. Composition for making up keratinous material, in the form of a wax-in-water emulsion, **characterized in that** it comprises at least one polyolefin wax resulting from the homopolymerization of alphaolefins having at least 10 carbon atoms, the said wax having a melting point ranging from 50 to 80°C.

2. Composition according to Claim 1, **characterized in that** the polyolefin wax has a melting point ranging from 50°C to 60°C.

3. Composition according to Claim 1 or 2, **characterized in that** the polyolefin wax has a needle penetration, measured at 44°C, which ranges from 15 to 120.

4. Composition according to any one of the preceding claims, **characterized in that** the polyolefin wax has a needle penetration, measured at 44°C, which ranges from 100 to 120 and, more preferably, from 105 to 115.

5. Composition according to any one of the preceding claims, **characterized in that** the said polyolefin wax has a number-average molecular weight ranging from 400 to 3000.

6. Composition according to any one of the preceding claims, **characterized in that** the said polyolefin wax has a number-average molecular weight ranging from 2000 to 3000 and, more preferably, from 2500 to 2700.

7. Composition according to any one of the preceding claims, **characterized in that** the said polyolefin wax has a density ranging from 0.85 to 0.95 g/cm³.

8. Composition according to any one of the preceding claims, **characterized in that** the polyolefin wax results from the homopolymerization of an alphaolefin corresponding to the general formula R-CH=CH₂ in which R denotes an alkyl radical having 10 to 50 carbon atoms or a mixture thereof.

9. Composition according to Claim 8, **characterized in that** R denotes an alkyl radical of 25 to 50 carbon atoms.

10. Composition according to either of Claims 8 and 9, **characterized in that** the alkyl radical R is linear.

11. Composition according to any one of the preceding claims, **characterized in that** the polyolefin wax is present in an amount ranging from 0.1% to 20% by weight relative to the total weight of the composition.

12. Composition according to any one of the preceding claims, **characterized in that** the polyolefin wax is present in an amount ranging from 0.5% to 12% by weight relative to the total weight of the composition.

13. Composition according to any one of the preceding claims, **characterized in that** it additionally comprises one or more additional waxes of animal, plant or synthetic origin.

14. Composition according to any one of the preceding claims, **characterized in that** the additional wax has a needle penetration at 25°C which ranges from 1 to 217.

15. Composition according to any one of the preceding claims, **characterized in that** it comprises at least one additional wax having a needle penetration ranging from 1 to 7.5 and at least one additional wax having a needle penetration of more than 7.5 and less than or equal to 217, measured at 25°C.

16. Composition according to one of Claims 13 to 15, **characterized in that** the additional wax is present in an amount ranging from 0.1% to 30% by weight relative to the total weight of the composition and, more preferably, from 1% to 20% by weight.

17. Composition according to any one of the preceding claims, **characterized in that** it additionally comprises at least one film-forming polymer.

18. Composition according to Claim 17, **characterized in that** the film-forming polymer is solubilized or in the form of particles in dispersion in the aqueous phase of the composition.

19. Composition according to either of Claims 17 and 18, **characterized in that** the amount of film-forming polymer ranges from 0.1% to 10% by weight relative to the total weight of the composition.

20. Composition according to any one of the preceding claims, **characterized in that** it comprises at least one emulsifying surfactant.

21. Composition according to Claim 20, **characterized in that** the emulsifying surfactant is present in an amount ranging from 2% to 30% by weight relative to the total weight of the composition.

22. Composition according to any one of the preceding claims, **characterized in that** it comprises at least one thickener.

23. Composition according to any one of the preceding claims, **characterized in that** the composition comprises at least one additive selected from the group consisting of vitamins, trace elements, softeners, sequestrants, perfumes, oils, silicones, vitamins, proteins, ceramides, plasticizers, cohesion agents, basifying or acidifying agents, fillers, pigments, emollients and preservatives.

24. Composition according to any one of the preceding claims, **characterized in that** the water content ranges from 20% to 99% by weight, preferably from 50% to 80% by weight, relative to the total weight of the composition.

25. Mascara product comprising a reservoir which contains a mascara composition and a brush for applying the said composition to keratinous fibres, especially the lashes, **characterized in that** the composition is a composition according to any one of Claims 1 to 24.

26. Method of making up keratinous material, especially the lashes, **characterized in that** a composition according to any one of Claims 1 to 24 is applied to the keratinous fibres.

27. Use of a composition as defined in any one of Claims 1 to 24 for obtaining a makeup which is homogeneous and/or easy to apply and/or which has a good hold.

28. Use of a polyolefin wax according to one of Claims 1 to 12 in a composition for making up keratinous fibres in the form of a wax-in-water emulsion.

29. Mascara comprising a composition according to any one of Claims 1 to 24.
